(19) Europäisches Patentamt / European Patent Office / Office européen des brevets

(11) **EP 3 338 778 A1**

(12) **EUROPEAN PATENT APPLICATION**
published in accordance with Art. 153(4) EPC


(43) Date of publication:
**27.06.2018 Bulletin 2018/26**

(21) Application number: **16837135.9**

(22) Date of filing: **17.08.2016**

(51) Int Cl.:
***A61K 31/47*** *(2006.01)* ***A61P 1/16*** *(2006.01)*
***A61P 35/00*** *(2006.01)* ***A61P 43/00*** *(2006.01)*

(86) International application number:
**PCT/JP2016/074017**

(87) International publication number:
**WO 2017/030146 (23.02.2017 Gazette 2017/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.08.2015 JP 2015162004**



(71) Applicant: **Eisai R&D Management Co., Ltd.**
**Tokyo 112-8088 (JP)**

(72) Inventors:
- **MATSUI, Junji**
  **Tsukuba-shi**
  **Ibaraki 300-2635 (JP)**
- **MATSUKI, Masahiro**
  **Tsukuba-shi**
  **Ibaraki 300-2635 (JP)**
- **TSURUOKA, Akihiko**
  **Tokyo 112-8088 (JP)**
- **TAMAI, Toshiyuki**
  **Tokyo 112-8088 (JP)**
- **NAKAJIMA, Ryo**
  **Tokyo 112-8088 (JP)**
- **SUZUKI, Takuya**
  **Tokyo 112-8088 (JP)**

(74) Representative: **Hoffmann Eitle**
**Patent- und Rechtsanwälte PartmbB**
**Arabellastraße 30**
**81925 München (DE)**


(54) **THERAPEUTIC AGENT FOR BILIARY TRACT CANCER**


(57) A therapeutic agent for biliary tract cancer comprising 4-[3-chloro-4-(cyclopropylaminocarbonyl)aminophenoxy]-7-methoxy-6-quinolinecarboxamide or a pharmacologically acceptable salt thereof is provided.


***Fig.1***

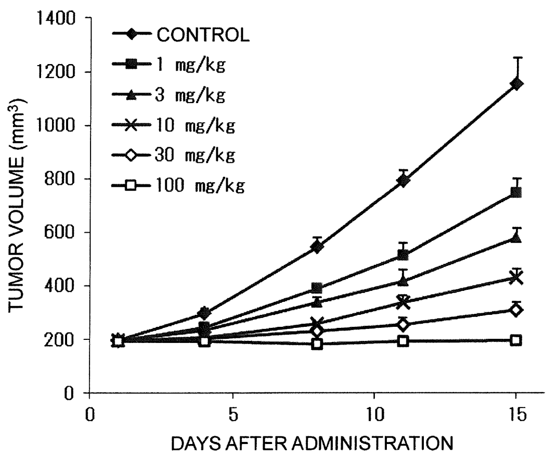


EP 3 338 778 A1

## Description

## Technical Field

The present invention relates to a therapeutic agent for biliary tract cancer comprising a compound having a kinase inhibitory effect. More specifically, the present invention relates to a therapeutic agent for biliary tract cancer comprising 4-[3-chloro-4-(cyclopropylaminocarbonyl)aminophenoxy]-7-methoxy-6-quinolinecarboxamide, which is a compound having a multi-tyrosine kinase inhibitory effect; or a pharmacologically acceptable salt thereof.

## Background Art

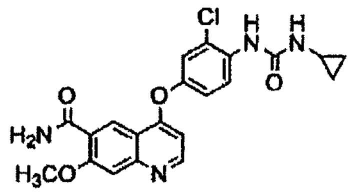

(I)

4-[3-chloro-4-(cyclopropylaminocarbonyl)aminophenoxy]-7-methoxy-6-quinolinecarboxamide represented by formula (I) (hereinafter referred to as compound A) has effects such as an antiangiogenic effect (Patent Literature 1) and an inhibitory effect (Patent Literatures 2 to 5) on a plurality of tyrosine kinases reported to be involved in malignant transformation of tumors (Non Patent Literatures 1 to 5). Methanesulfonate (mesylate) of compound A has been approved as a drug for treating thyroid cancer and renal cell carcinoma, and also has been in clinical trials for various tumors such as lung cancer, melanoma, endometrial cancer, glioma, hepatocellular carcinoma, and ovarian cancer.

Biliary tract cancer is a cancer of an intrahepatic bile duct, an extrahepatic bile duct, a cystic duct, a gallbladder, or a ampulla of Vater and its incidence rate is low; however, it is known as a tumor with poor prognosis. The definitive treatment method of biliary tract cancer is extirpation by surgery; however, there are few cases in which biliary tract cancers are operable at the time of being diagnosed, and there are many cases in which biliary tract cancers cannot be extirpated completely even if they are operable. In inoperable cases, combined administration of gemcitabine and cisplatin has been used (Non Patent Literatures 6 and 7).

## Citation List

### Patent Literature

Patent Literature 1: United States Patent Publication No. 2004-053908
Patent Literature 2: United States Patent Publication No. 2004-253205
Patent Literature 3: United States Patent Publication No. 2010-105031
Patent Literature 4: United States Patent Publication No. 2009-209580
Patent Literature 5: United States Patent Publication No. 2009-264464


### Non Patent Literature

Non Patent Literature 1: Lasota et al., "Mutations in Exons 9 and 13 of KIT Gene Are Rare Events in Gastrointestinal Stromal Tumors", American Journal of Pathology, vol.157, p.1091-1095, 2000.

Non Patent Literature 2: Berdel et al., "Recombinant Human Stem Cell Factor Stimulates Growth of a Human Glioblastoma Cell Line Expressing c-kit Protooncogenel", Cancer Research, vol.52, p.3498-3502, 1992.

Non Patent Literature 3: Lennartsson et al., "The stem cell factor receptor/c-Kit as a drug target in cancer", Current Cancer Drug Targets, vol.6, p.65-75, 2006.

Non Patent Literature 4: Turner et al., "Fibroblast growth factor signalling: from development to cancer", Nature Reviews Cancer, vol.10, p.116-129, 2010.

Non Patent Literature 5: Wells et al., "Targeting the RET Pathway in Thyroid Cancer", Clinical Cancer Research, vol.15, p.7119-7123, 2009.
Non Patent Literature 6: Juan et al., "Cisplatin plus gemcitabine versus gemcitabine for biliary tract cancer", The New England Journal of Medicine, voL362, p.1273-1281,2010.
Non Patent Literature 7: Ang, "Role of the fibroblast growth factor receptor axis in cholangiocarcinoma", Journal of Gastroenterology and Hepatology, vol.30, p.1116-1122, 2015.

**Summary of Invention**

**Technical Problem**

However, the therapeutic effect of a therapeutic agent for biliary tract cancer reported to date is not sufficient, and development of a further new therapeutic agent has been awaited.

**Solution to Problem**

In view of the above circumstances, the present inventors conducted intensive research, and consequently found compound A to exhibit a highly therapeutic effect on biliary tract cancer and completed the present invention.
Thus, the present invention provides the following [1] to [17]:

[1] A therapeutic agent for biliary tract cancer comprising compound A or a pharmacologically acceptable salt thereof.
[2] A pharmaceutical composition for treating biliary tract cancer comprising compound A or a pharmacologically acceptable salt thereof.
[3] A method for treating biliary tract cancer, comprising administering compound A or a pharmacologically acceptable salt thereof to a patient in need thereof.
[4] The above-mentioned method further comprising administering at least one antitumor agent to the patient.
[5] Compound A or a pharmacologically acceptable salt thereof for treating biliary tract cancer.
[6] A pharmaceutical composition comprising compound A or a pharmacologically acceptable salt thereof for treating biliary tract cancer.
[7] Use of compound A or a pharmacologically acceptable salt thereof, for treating biliary tract cancer.
[8] Use of compound A or a pharmacologically acceptable salt thereof for the manufacture of a therapeutic agent for biliary tract cancer or a pharmaceutical composition for treating biliary tract cancer.
[9] The above-mentioned therapeutic agent or pharmaceutical composition further comprising an excipient.
[10] The above-mentioned therapeutic agent, pharmaceutical composition, treatment method, use, or compound wherein compound A or pharmacologically acceptable salt thereof is methanesulfonate of compound A.
[11] The above-mentioned therapeutic agent, pharmaceutical composition, treatment method, use, or compound wherein the biliary tract cancer is intrahepatic cholangiocarcinoma, extrahepatic cholangiocarcinoma, cystic duct adenocarcinoma, gallbladder cancer, or carcinoma of the ampulla of Vater.
[12] The above-mentioned therapeutic agent, pharmaceutical composition, treatment method, use, or compound wherein the biliary tract cancer is intrahepatic cholangiocarcinoma.
[13] The above-mentioned therapeutic agent, pharmaceutical composition, treatment method, use, or compound wherein the biliary tract cancer is extrahepatic cholangiocarcinoma.
[14] The above-mentioned therapeutic agent, pharmaceutical composition, treatment method, use, or compound wherein the biliary tract cancer is cystic duct adenocarcinoma.
[15] The above-mentioned therapeutic agent, pharmaceutical composition, treatment method, use, or compound wherein the biliary tract cancer is gallbladder adenocarcinoma.
[16] The above-mentioned therapeutic agent, pharmaceutical composition, treatment method, use, or compound wherein the biliary tract cancer is carcinoma of the ampulla of Vater.
[17] The above-mentioned therapeutic agent, pharmaceutical composition, treatment method, use, or compound, wherein compound A or a pharmacologically acceptable salt thereof is administered orally at a dosage of 1 to 100 mg per day.

**Advantageous Effects of Invention**

The present invention provides the therapeutic agent for biliary tract cancer comprising compound A having a multi-tyrosine kinase inhibitory effect The effect of compound A on reducing tumor volume may be examined by administering compound A to a tumor model of biliary tract cancer or a biliary tract cancer patient by the method described in Examples.

**Brief Description of Drawings**

Figure 1 is a graph showing an antitumor effect of compound A on a model subcutaneously transplanted with a human extrahepatic cholangiocarcinoma cell line.

Figure 2 is a graph showing an average tumor volume on Day 15 for each group of the models subcutaneously transplanted with the human extrahepatic cholangiocarcinoma cell line.

Figure 3 is a graph showing an antitumor effect of compound A on a model subcutaneously transplanted with a human cholangiocarcinoma cell line.

Figure 4 is a graph showing an average tumor volume on Day 15 for each group of the models subcutaneously transplanted with the human cholangiocarcinoma cell line.

Figure 5 is a graph showing an antitumor effect of compound A on a model subcutaneously transplanted with a human carcinoma of the ampulla of Vater cell line.

Figure 6 is a graph showing an average tumor volume on Day 15 for each group of the models subcutaneously transplanted with the human carcinoma of the the ampulla of Vater cell line.

**Description of Embodiments**

Hereinbelow, the embodiments of the present invention will be described. The following embodiments are illustrated to describe the present invention and are not intended to limit the present invention to these embodiments. The present invention may be carried out in various modes without departing from the subject matter of the present invention.

The present application claims priority to Japanese Patent Application No. 2015-162004 filed in Japan. Literatures, and publications of unexamined applications, patent publications, and other patent literatures cited in the description are herein incorporated by reference.

Compound A or a pharmacologically acceptable salt thereof of the present invention can be produced by the method described in Patent Literature 1. An example of the pharmacologically acceptable salt of compound A is methanesulfonate (mesylate).

Examples of the pharmacologically acceptable salt include, but are not limited to a specific type of salts, salts with inorganic acids, salts with organic acids, salts with inorganic bases, salts with organic bases, and salts with acidic amino acids or basic amino acids.

Examples of the salts with inorganic acids include, but are not limited to a specific type of salts, salts with hydrochloric acid, hydrobromic acid, sulphuric acid, nitric acid, or phosphoric acid. Examples of the salts with organic acids include, but are not limited to a specific type of salts, salts with acetic acid, succinic acid, fumaric acid, maleic acid, tartaric acid, citric acid, lactic acid, stearic acid, benzoic acid, methane sulfonic acid (mesylate), ethane sulfonic acid, or p-toluenesulfonic acid.

Examples of the salts with inorganic bases include, but are not limited to a specific type of salts, alkali metal salts such as a sodium salt and a potassium salt; alkaline earth metal salts such as a calcium salt and a magnesium salt; an aluminum salt, and an ammonium salt Examples of the salts with organic bases include, but are not limited to a specific type of salts, salts with diethylamine, diethanolamine, meglumine, or N,N-dibenzylethylenediamine.

Examples of the salts with acidic amino acids include, but are not limited to a specific type of salts, salts with aspartic acid or glutamic acid. Examples of the salts with basic amino acids include, but are not limited to a specific type of salts, salts with arginine, lysine, or ornithine.

One aspect of the pharmacologically acceptable salt of compound A is methanesulfonate (mesylate).

The dosage of compound A or the pharmacologically acceptable salt thereof can be selected as appropriate depending on the severity of the symptom, the age, gender, weight, and sensitivity difference of the patient, the administration route, the timing of administration, the dosing interval, the type of the pharmaceutical formulation, and the like. Usually, the dosage is 0.1 to 500 mg/day, preferably 0.5 to 300 mg/day, more preferably 1.0 to 100 mg/day when orally administered to an adult (body weight: 60 kg). This dosage may be administered once a day or may be divided and administered in two or three portions. One aspect of the dosage of compound A or the pharmacologically acceptable salt thereof when orally administered to an adult (body weight: 60 kg) is 24 mg per day in terms of compound A in free fonn.

The therapeutic agent of the present invention can be formulated by a method described in, for example, the Japanese Pharmacopoeia 16th Edition (JP), the United States Pharmacopeia (USP), or the European Pharmacopoeia (EP).

The therapeutic agent of the present invention can be administered orally in the form of a solid formulation such as a tablet, a granule, a fine granule, a powder, and a capsule, or a liquid formulation, a jelly, a syrup, or the like.

The therapeutic agent of the present invention may also be administered parenterally in the form such as an

injection, a suppository, an ointment, and a cataplasm.

For preparation of an oral solid formulation, an excipient, and furthermore, if necessary, a binder, a disintegrant, a lubricant, a colorant, a flavoring agent, and the like are added as an additive to the active pharmaceutical ingredient, which is compound A or the pharmacologically acceptable salt thereof Then, for example, a tablet, a granule, a fine granule, a powder, and a capsule can be formulated by a conventional method. The above-mentioned additives can be combined as appropriate for formulation. Coating may also be applied to the tablet, the granule, and the like if necessary.

Examples of the excipient include lactose, sucrose, glucose, com starch, mannitol, sorbitol, starch, pregelatinized starch, dextrin, microcrystalline cellulose, and calcium hydrogen phosphate.

Examples of the binder include methylcellulose, ethylcellulose, gum arabic, hydroxypropylmethylcellulose, and hydroxypropylcellulose.

Examples of the disintegrant include low-substituted hydroxypropylcellulose, carboxymethylcellulose, carboxymethylcellulose calcium, croscarmellose sodium, sodium carboxymethyl starch, and crospovidone.

Examples of the lubricant include talc, silica, magnesium stearate, calcium stearate, sodium stearyl fumarate, and polyethyleneglycol.

Examples of the colorant include iron sesquioxide, yellow iron sesquioxide, carmine, β-carotene, titanium oxide, riboflavin sodium phosphate, yellow aluminum lake, and cochineal.

Examples of the flavoring agent include cocoa powder, ascorbic acid, tartaric acid, peppermint oil, borneol, and cinnamon powder.

For preparation of an injection, a pH adjustor, a buffering agent, a suspending agent, a solubilizer, a stabilizing agent, an isotonic agent, a preservative, and the like are added to the active pharmaceutical ingredient if necessary; and an intravenous, subcutaneous, or intramuscular injection, or an intravenous infusion can be formulated by a conventional method. If necessary, such injections may be formulated as a lyophilizate by a conventional method.

Examples of the pH adjustor and the buffering agent include hydrochloric acid, sodium carbonate, sodium hydrogen carbonate, citric acid, sodium citrate, sodium dihydrogen citrate, glycine, phosphoric acid, sodium dihydrogen phosphate, sodium hydrogen phosphate, sodium hydroxide, acetic acid, sodium acetate, and meglumine.

Examples of the suspending agent include sodium alginate, sucrose fatty acid ester, polysorbate 80, gum arabic, powdered tragacanth, and polyoxyethylene sorbitan monolaurate.

Examples of the solubilizer include polyoxyethylene hydrogenated castor oil, polysorbate 80, nicotinamide, polyoxyethylene sorbitan monolaurate, glycerine fatty acid ester, polyethylene glycol, propylene glycol, benzyl benzoate, ethanol, and triethanolamine.

Examples of the stabilizing agent include sodium sulfite and sodium metasulfite.

Examples of the isotonic agent include glucose, mannitol, and sorbitol.

Examples of the preservative include methyl parahydroxybenzoate, ethyl parahydroxybenzoate, sorbic acid, phenol, cresol, and chlorocresol.

As used herein, "biliary tract cancer" refers to intrahepatic cholangiocarcinoma, extrahepatic cholangiocarcinoma, cystic duct adenocarcinoma, gallbladder adenocarcinoma, or carcinoma of the ampulla of Vater. Intrahepatic cholangiocarcinoma and extrahepatic cholangiocarcinoma are collectively referred to as cholangiocarcinoma. Cancers resulting from metastasis of such cancers to a part other than the biliary tract are encompassed by the above-mentioned biliary tract cancer.

## Examples

Hereinbelow, the present invention is illustrated by specific examples; however, the present invention is not limited to these examples.

[Example 1] A tumor growth-inhibitory effect of compound A on a model subcutaneously transplanted with a human extrahepatic cholangiocarcinoma cell line (TFK-1 cell line)

An antitumor effect produced by administration of compound A was evaluated with six nude mice in each group (CAnN.Cg-Foxn1$^{nu}$/CrlCrlj, female, Charles River Laboratories Japan, Inc.). The human-derived extrahepatic cholangiocarcinoma TFK-1 cell lines (Riken BioResource Center) were suspended in RPMI1640 medium (Wako Pure Chemical Industries, Ltd., supplemented with 10% FBS) at a concentration of $2 \times 10^8$ cells/mL and were mixed well. The cell suspension was further mixed with an equal amount of Matrigel basement membrane matrix (Coming Incorporated) to prepare a cell suspension at $1 \times 10^8$ cells/mL, and aliquots of 0.1 mL were subcutaneously transplanted into the right flank of each mouse. Seven days after transplantation, the longest diameter and the short axis of the tumors were measured by an electronic digital caliper (ABS Digimatic Caliper, Mitutoyo Corporation). The mice were divided into groups so that the average of tumor volumes of each group became approximately equal. In this regard, the tumor volume was calculated according to the following formula.

$$\text{Tumor volume (mm}^3\text{)} = \text{longest diameter (mm)} \times \text{short axis (mm)} \times \text{short axis (mm)}/2$$

Mesylate of compound A was dissolved in 3 mmol/L hydrochloric acid solution so that the concentrations became 0.05, 0.15, 0.5,1.5, and 5 mg/mL.

A control group and compound A groups (1, 3, 10,30, and 100 mg/kg) were set up. Three mmol/L hydrochloric acid solution and 3 mmol/L hydrochloric acid solutions comprising the respective amount of mesylate of compound A were administered orally at a dose of 20 mL/kg once a day, to the control group and the compound A groups, respectively. The administration period was set to 14 days.

The tumor volume was measured on the day when the administration was started (Day 1), Day 4, Day 8, and Day 11, and the day following the last day of administration (Day 15). The change of the tumor volume over time was shown in Figure 1 and the average of tumor volumes of each group on Day 15 was shown in Figure 2. Furthermore, comparison between the control group and the compound A groups was made based on the tumor volume on Day 15 with Dunnett's multiple test, and P-values less than 0.05 were regarded as significant difference. Consequently, it was found that compound A had a dose-dependent antitumor effect in the model subcutaneously transplanted with the human extrahepatic cholangiocarcinoma-derived cell line (TFK-1 cell line) (Figures 1 and 2). **** in Figure 2 indicates that tumor growth was statistically significantly inhibited (P-value less than 0.0001).

[Example 2] A tumor growth-inhibitory effect of compound A on a model subcutaneously transplanted with a human cholangiocarcinoma cell line (OZ cell line)

An antitumor effect produced by administration of compound A was evaluated with five nude mice in each group (CAnN.Cg-Foxn1$^{nu}$/CrlCrlj, female, Charles River Laboratories Japan, Inc.). The human-derived cholangiocarcinoma OZ cell lines (JCRB Cell Bank) were suspended in Williams'E (Sigma-Aldrich Corporation, supplemented with 2 mmol/L L-glutamine and 10% FBS) at a concentration of $2 \times 10^8$ cells/mL and were mixed well. The cell suspension was further mixed with an equal amount of Matrigel basement membrane matrix (Coming Incorporated) to prepare a cell suspension at $1 \times 10^8$ cells/mL, and aliquots of 0.1 mL were subcutaneously transplanted into the right flank of each mouse. Thirteen days after transplantation, the longest diameter and the short axis of the tumors were measured by an electronic digital caliper (ABS Digimatic Caliper, Mitutoyo Corporation). The mice were divided into groups so that the average of tumor volumes of each group became approximately equal. In this regard, the tumor volume was calculated according to the following formula.

$$\text{Tumor volume (mm}^3\text{)} = \text{longest diameter (mm)} \times \text{short axis (mm)} \times \text{short axis (mm)}/2$$

Mesylate of compound A was dissolved in 3 mmol/L hydrochloric acid solution so that the concentrations became 0.05, 0.15, 0.5, 1.5, and 5 mg/mL.

A control group and compound A groups (1,3,10,30, and 100 mg/kg) were set up. Three mmol/L hydrochloric acid solution and 3 mmol/L hydrochloric acid solutions comprising the respective amount of mesylate of compound A were administered orally at a dose of 20 mL/kg once a day, to the control group and the compound A groups, respectively. The administration period was set to 14 days.

The tumor volume was measured on the day when the administration was started (Day 1), Day 5, Day 8, and Day 12, and the day following the last day of administration (Day 15). The change of the tumor volume over time was shown in Figure 3 and the average of tumor volumes of each group on Day 15 was shown in Figure 4. Furthermore, comparison between the control group and the compound A groups was made based on the tumor volume on Day 15 with Dunnett's multiple test and P-values less than 0.05 were regarded as significant difference. Consequently, it was found that compound A had a dose-dependent antitumor effect in the model subcutaneously transplanted with the human cholangiocarcinoma-derived cell line (OZ cell line) (Figures 3 and 4). ****in Figure 4 indicates that tumor growth was statistically significantly inhibited (P-value less than 0.0001).

[Example 3] A tumor growth-inhibitory effect of compound A on a model subcutaneously transplanted with a human carcinoma of the ampulla of Vater cell line (SNU-869 cell line)

An antitumor effect produced by administration of compound A was evaluated with six nude mice in each group (CAnN.Cg-Foxn1$^{nu}$/CrlCtrlj, female, Charles River Laboratories Japan, Inc.). The human-derived human carcinoma of the ampulla of Vater SNU-869 cell lines (Creative Bioarray) were suspended in RPMI1640 medium (Wako Pure Chemical Industries, Ltd., supplemented with 25mM HEPES and 10% FBS) at a concentration of 2 x $10^8$ cells/mL and were mixed

well. The cell suspension was further mixed with an equal amount of Matrigel basement membrane matrix (Coming Incorporated) to prepare a cell suspension at 1 $\times$ $10^8$ cells/mL, and aliquots of 0.1 mL were subcutaneously transplanted into the right flank of each mouse. Seven days after transplantation, the longest diameter and the short axis of the tumors were measured by an electronic digital caliper (ABS Digimatic Caliper, Mitutoyo Corporation). The mice were divided into groups so that the average of tumor volumes of each group became approximately equal. In this regard, the tumor volume was calculated according to the following formula.

$$\text{Tumor volume (mm}^3\text{)} = \text{longest diameter (mm)} \times \text{short axis (mm)} \times \text{short axis (mm)}/2$$

Mesylate of compound A was dissolved in 3 mmol/L hydrochloric acid solution so that the concentrations became 0.05, 0.15, 0.5,1.5, and 5 mg/mL.

A control group and compound A groups (1,3,10,30, and 100 mg/kg) were set up. Three mmol/L hydrochloric acid solution and 3 mmol/L hydrochloric acid solutions comprising the respective amount of mesylate of compound A were administered orally at a dose of 20 mL/kg once a day, to the control group and the compound A groups, respectively. The administration period was set to 14 days.

The tumor volume was measured on the day when the administration was started (Day 1), Day 4, Day 8, and Day 11, and the day following the last day of administration (Day 15). The change of the tumor volume over time was shown in Figure 5 and the average of tumor volumes of each group on Day 15 was shown in Figure 6. Furthermore, comparison between the control group and the compound A groups was made based on the tumor volume on Day 15 with Dunnett's multiple test and P-values less than 0.05 were regarded as significant difference. Consequently, it was found that compound A had a dose-dependent antitumor effect in the model subcutaneously transplanted with the human carcinoma of the ampulla of Vater cell line (SNU-869 cell line) (Figures 5 and 6). ****in Figure 6 indicates that tumor growth was statistically significantly inhibited (P-value less than 0.0001).

[Example 4] A multicenter, unblinded, Phase II clinical trial of compound A for patients with an unresectable biliary tract cancer after combination chemotherapy involving gemcitabine

This clinical trial was a multicenter, single-group, unblinded clinical trial for patients with a prior treatment history who had received one regimen using two-drug combination chemotherapy involving gemcitabine (two-drug combination chemotherapy using gemcitabine and cisplatin, or gemcitabine and another platinum-based drug or a fluorinated pyrimidine drug) for their unresectable biliary tract cancers.

This clinical trial was composed of three periods, which were a pretreatment period, an administration period, and a follow-up period. During the pretreatment period, screening was performed within 21 days. The administration period was composed of administration of mesylate of compound A as an investigational drug in each cycle and tumor assessment performed every 6 to 8 weeks. The follow-up period was started immediately after the day when administration was discontinued and was continued while the subject was alive until withdrawal of consent by the subject or completion of the clinical triaL

The investigational drug, mesylate of compound A, was administered orally once a day in a cycle of 28 days at a dose of 24 mg in terms of compound A in free form, and administration was continued until disease progression, an unacceptable adverse event, withdrawal of consent by the subject, or the like occurred.

The primary objective of the clinical trial was to determine an objective response rate (ORR) of the investigational drug for patients with an unresectable biliary tract cancer after combination chemotherapy involving gemcitabine.

The secondary objective of the clinical trial was to determine a progression-free survival (PFS), an overall survival (OS), a disease control rate (DCR), a clinical benefit rate (CBR), adverse events (AEs), and the number of subjects who developed serious adverse events (SAEs), and a blood concentration. In this context, ORR is defined as a rate of subjects with their best overall response (BOR) being complete response (CR) or partial response (PR). PFS is defined as a period from the first administration day of the investigational drug to the day when an event (either disease progression or death regardless of the cause of death, whichever is earlier) was observed for the first time. OS is defined as a period from the first administration day of the investigational drug to the day of death regardless of the cause of death. DCR is defined as a percentage of the subjects with CR, PR, or stable disease (SD). CBR is defined as a percentage of the subjects with CR, PR, or long-term SD. Long-term SD is defined as a SD lasting for 23 weeks or longer.

Subjects fulfilling all of the following criteria were included in the clinical trial.

(1) Patients with a biliary tract cancer diagnosed histologically or cytologically with adenocarcinoma (intrahepatic cholangiocarcinoma, extrahepatic cholangiocarcinoma, gallbladder adenocarcinoma, or carcinoma of the ampulla of Vater)

(2) Patients with an unresectable (for example, locally advanced or metastatic) biliary tract cancer
(3) Patients who have received one regimen as prior treatment for their unresectable biliary tract cancers, the regimen using two-drug combination chemotherapy involving gemcitabine (for example, gemcitabine and cisplatin), and have not received any other chemotherapy for biliary tract cancer
In this regard, patients who have received postoperative adjuvant chemotherapy are deemed eligible if the therapy is completed and no recurrence has been observed for six months after completion of the therapy.
(4) Patients with measurable lesions fulfilling the following criteria:
Patients have at least one continuously measurable lesion with the longest diameter being 1.0 cm or more in the case of a non-lymph node or with the short axis being 1.5 cm or more in the case of a lymph node, as assessed by CT or MRI based on Response Evaluation Criteria in Solid Tumors 1.1 (RECIST1.1).
When a lesion previously subjected to local treatment such as external-beam radiation therapy (EBRT) or radiofrequency (RF) ablation is a target lesion, disease progression according to RECIST1.1 has been observed.
(5) Patients with Eastern Cooperative Oncology Group (ECOG) Performance Status of 0 to 1
(6) Patients with a life expectancy of three months or more, after initiation of administration of the investigational drug
(7) Men or women aged 20 years or older at the time of obtaining consent
(8) Patients in whom all the toxicities associated with chemotherapy or radiotherapy other than hair loss, infertility, and adverse events within the inclusion criteria have recovered to Grade 0 to 1 according to Common Terminology Criteria for Adverse Events (CTCAE v4.03)
(9) Patients with well-controlled blood pressure (BP) with or without the use of an antihypertensive (definition: systolic blood pressure is 150 mmHg or less and diastolic blood pressure is 90 mmHg or less, and a change of the antihypertensive has not been made within one week prior to initiation of administration of the investigational drug)
(10) Patients whose function of the major organ and anticoagulant ability are sufficiently preserved

1) Neutrophil count (ANC) $\geq 1500/mm^3$ ($\geq 1.5 \times 10^3/\mu L$)
2) Platelet count $\geq 100,000/mm^3$ ($\geq 100 \times 10^9/L$)
3) Hemoglobin $\geq$ 9.0 g/dL
4) Total bilirubin $\leq$ 2.0 mg/dL (excluding unconjugated hyperbilirubinemia or Gilbert syndrome)
5) ALP, AST, and ALT $\leq 3.0 \times$ ULN (the upper limit of local laboratory reference value) ($\leq 5.0 \times$ ULN when the patient has liver metastasis)
6) Creatinine clearance $\geq$ 40 mL/min (Cockcroft-Gault method)
7) Prothrombin time-international normalized ratio (PT-INR) $\leq$ 1.5

(11) Patients who consented to participate in the clinical trial voluntarily in a written form
(12) Patients willing to comply with the clinical trial protocol and capable of complying with it

Subjects fulfilling any of the following criteria were excluded from the clinical trial.

(1) Patients who have received anticancer treatment (other than BSC) within 21 days prior to initiation of administration of the investigational drug
(2) Patients who have undergone a major operation (an operation requiring anesthesia or assisted respiration) within 21 days prior to initiation of administration of the investigational drug, or patients whose operation is planned to be performed during the clinical trial (excluding biliary drainage)
(3) Patients with moderate-grade ascites, high-grade ascites, or ascites requiring drainage
(4) Patients with proteinuria of 2+ or more on dipstick (when a grade determined by quantitative evaluation is Grade 1 or lower, the patient is judged eligible)
(5) Patients exhibiting malabsorption in the gastrointestinal tract or other physical conditions that are judged likely to interfere with absorption of the investigational drug by an investigator or a subinvestigator
(6) Patients who have had congestive heart failure of class 2 or worse by New York Heart Association classification, unstable angina pectoris, myocardial infarction, or serious arrhythmia associated with a clinically significant cardiovascular disorder within six months prior to initiation of administration of the investigational drug
(7) Patients with QT/QTc interval prolongation (QTcF > 480ms)
(8) Patients found to be positive for an HIV antibody test
(9) Patients with an active infection requiring systemic therapy
(10) Patients suffering from a hemorrhagic or thrombotic disease, or chronically using an anticoagulant such as warfarin or a pharmaceutical agent of the same kind requiring INR monitoring (use of low molecular weight heparin is permitted)
(11) Patients in whom gastrointestinal bleeding or active hemoptysis (a half teaspoon or more of bright red blood) was observed within 21 days prior to initiation of administration of the investigational drug

(12) Patients who have had an active malignant tumor within 24 months prior to initiation of administration of the investigational drug (excluding biliary tract cancer, completely treated noninvasive melanoma, basal cell carcinoma or squamous cell carcinoma of the skin, noninvasive cervical cancer, and early stomach cancer/large bowel cancer)
(13) Patients diagnosed with meningeal carcinomatosis
(14) Patients with brain metastasis or subdural metastasis
However, an exception is made for patients in whom topical treatment has been already completed and administration of adrenocortical hormone for treatment of the metastasis has been discontinued 28 days or more prior to initiation of administration of the investigational drug. Signs (for example, by radiographic examination) or symptoms of brain metastasis must be stable 28 days or more prior to initiation of administration of the investigational drug.
(15) Patients found unable to tolerate the investigational drug or any ingredient of the excipients
(16) Patients with a history of drug or alcohol dependence or abuse within 24 months prior to initiation of administration of the investigational drug
(17) Patients judged by an investigator or a subinvestigator ineligible as a subject participating in the clinical trial due to medical or other reasons
(18) If a patient is a woman, patients lactating or pregnant during screening or at baseline (patients identified as positive by human chorionic gonadotropin (hCG or β-hCG) test)
When the test during screening was carried out three or more days prior to initiation of administration of the investigational drug, a retest needs to be performed even if the test result was negative.
(19) If a patient is a reproductive man or a woman of childbearing potential, patients who do not or whose partners do not consent to use a medically suitable contraception method during the course of the clinical trial

For the above-mentioned contraception method, a condom, a contraceptive sponge, a contraceptive foam, a contraceptive jelly, a pessary, or an intrauterine device must be used, or an oral contraceptive (a transdermal or transvaginal contraceptive may be used) must be used from 28 days or more prior to initiation of administration of the investigational drug.

Result of interim analysis

An interim analysis of the clinical study was performed. The total number of patients included in the interim analysis were 17, and 3 of them were patients with extrahepatic cholangiocarcinoma, 4 of them were patients with intrahepatic cholangiocarcinoma, 9 of them were patients with gallbladder adenocarcinoma, and one of them was a patient with carcinoma of the ampulla of Vater.
The result of the interim analysis showed partial response (PR) in one patient (5.9%) and stable disease (SD, 6 weeks or more) in 13 patients (76.5%). Furthermore, tendency for tumor regression was observed in 8 patients among 13 SD patients.

## Claims

1. A therapeutic agent for biliary tract cancer comprising 4-[3-chloro-4-(cyclopropylaminocarbonyl)aminophenoxy]-7-methoxy-6-quinolinecarboxamide or a pharmacologically acceptable salt thereof.

2. A pharmaceutical composition for treating biliary tract cancer comprising 4-[3-chloro-4-(cyclopropylaminocarbonyl)aminophenoxy]-7-methoxy-6-quinolinecarboxamide or a pharmacologically acceptable salt thereof.

3. The therapeutic agent according to claim 1 or the pharmaceutical composition according to claim 2, wherein the pharmacologically acceptable salt is methanesulfonate.

4. The therapeutic agent or the pharmaceutical composition according to any one of claims 1 to 3 further comprising an excipient

5. The therapeutic agent or the pharmaceutical composition according to any one of claims 1 to 4, wherein the biliary tract cancer is intrahepatic cholangiocarcinoma, extrahepatic cholangiocarcinoma, cystic duct adenocarcinoma, gallbladder adenocarcinoma, or carcinoma of the ampulla of Vater.

6. The therapeutic agent or the pharmaceutical composition according to claim 5, wherein the biliary tract cancer is intrahepatic cholangiocarcinoma.

7. The therapeutic agent or the pharmaceutical composition according to claim 5, wherein the biliary tract cancer is extrahepatic cholangiocarcinoma.

8. The therapeutic agent or the pharmaceutical composition according to claim 5, wherein the biliary tract cancer is cystic duct adenocarcinoma.

9. The therapeutic agent or the pharmaceutical composition according to claim 5, wherein the biliary tract cancer is gallbladder adenocarcinoma.

10. The therapeutic agent or the pharmaceutical composition according to claim 5, wherein the biliary tract cancer is carcinoma of the ampulla of Vater.

11. The therapeutic agent or the pharmaceutical composition according to any one of claims 1 to 10, wherein 4-[3-chloro-4-(cyclopropylaminocarbonyl)aminophenoxy]-7-methoxy-6-quinolinecarboxamide or the pharmacologically acceptable salt thereof is administered orally at a dosage of 1 to 100 mg per day.

12. A method for treating biliary tract cancer, comprising administering 4-[3-chloro-4-(cyclopropylaminocarbonyl)aminophenoxy]-7-methoxy-6-quinolinecarboxamide or the pharmacologically acceptable salt thereof to a patient in need thereof

13. The method according to claim 12, further comprising administering at least one antitumor agent to the patient

14. A pharmaceutical composition comprising 4-[3-chloro-4-(cyclopropylaminocarbonyl)aminophenoxy]-7-methoxy-quinolinecarboxamide or a pharmacologically acceptable salt thereof for treating biliary tract cancer.

15. Use of 4-[3-chloro-4-(cyclopropylaminocarbonyl)aminophenoxy]-7-methoxy-6-quinolinecarboxamide or a pharmacologically acceptable salt thereof, for the manufacture of a pharmaceutical composition for treating biliary tract cancer.

*Fig.1*

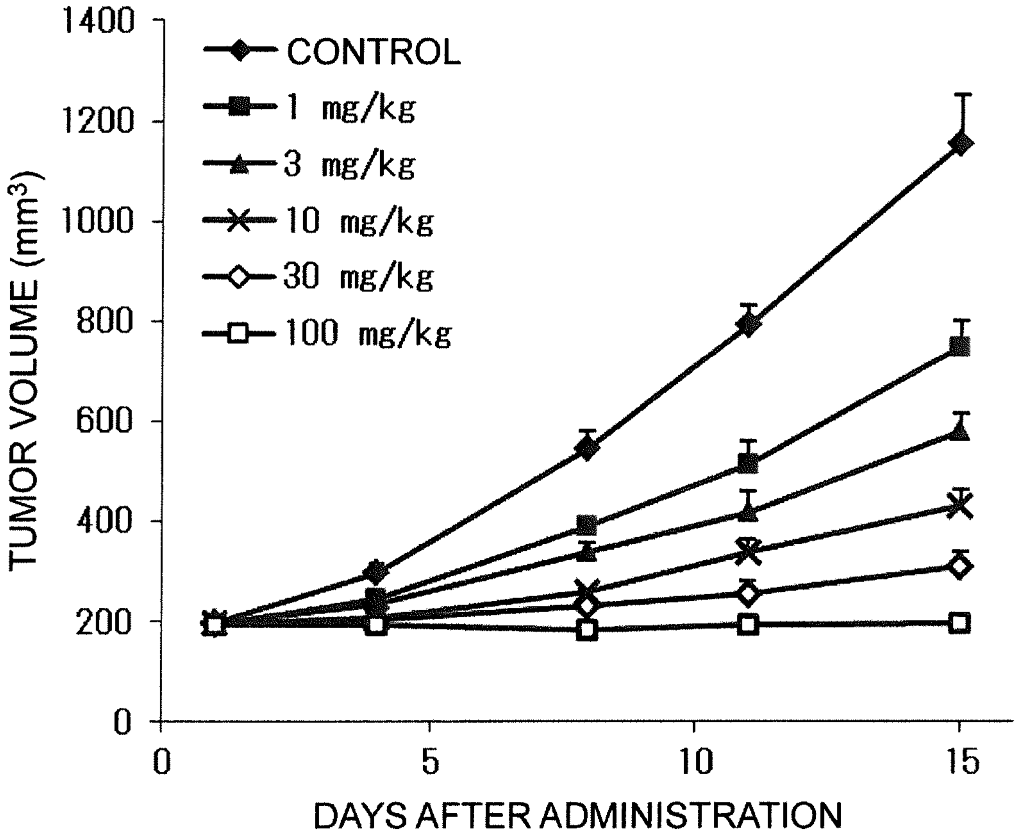
1400
1200
1000
800
600
400
200
0
TUMOR VOLUME (mm3)
CONTROL
1 mg/kg
3 mg/kg
10 mg/kg
30 mg/kg
100 mg/kg
0
5
10
15
DAYS AFTER ADMINISTRATION

# Fig.2

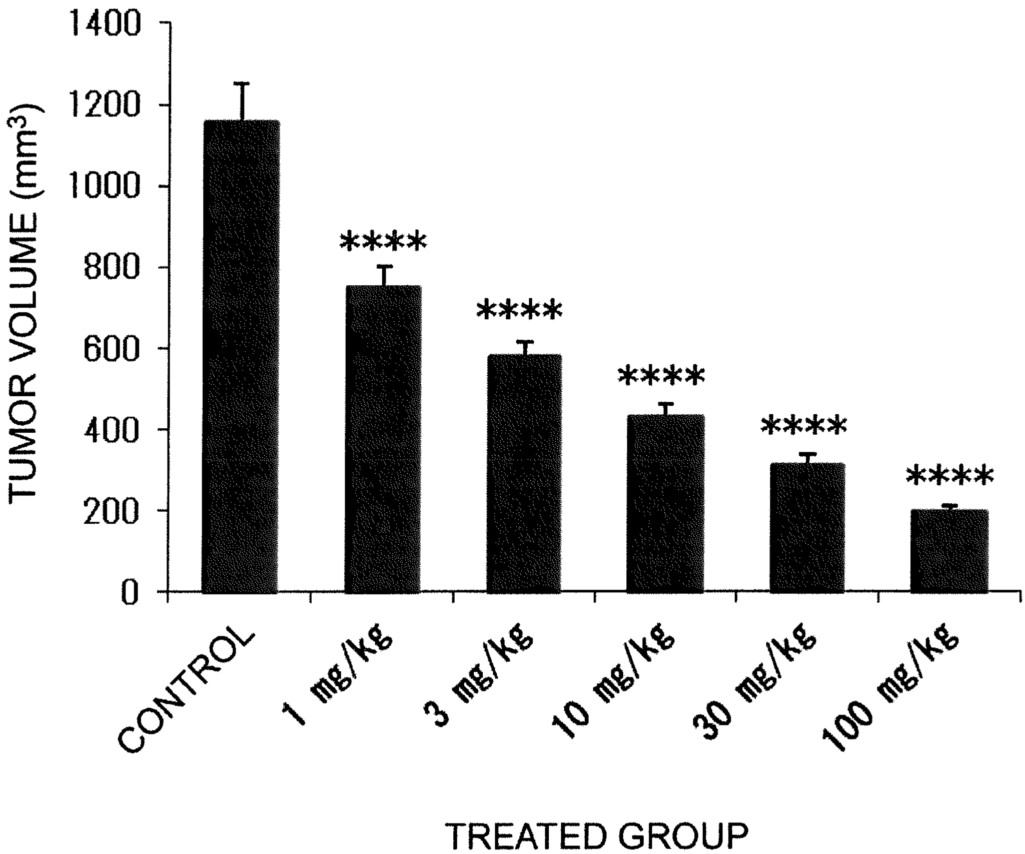
1400
1200
1000
800
600
400
200
0
TUMOR VOLUME (mm3)
****
****
****
****
****
CONTROL
1 mg/kg
3 mg/kg
10 mg/kg
30 mg/kg
100 mg/kg
TREATED GROUP

Fig.3

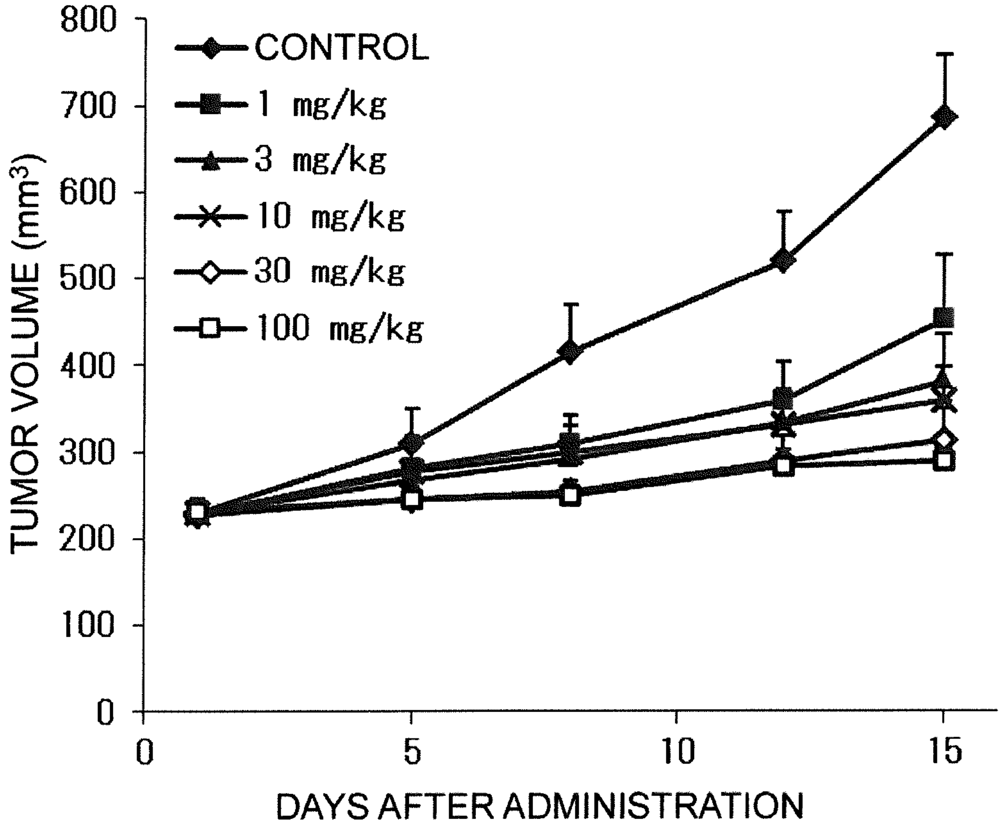
CONTROL
1 mg/kg
3 mg/kg
10 mg/kg
30 mg/kg
100 mg/kg
TUMOR VOLUME (mm3)
800
700
600
500
400
300
200
100
0
0
5
10
15
DAYS AFTER ADMINISTRATION

*Fig.4*

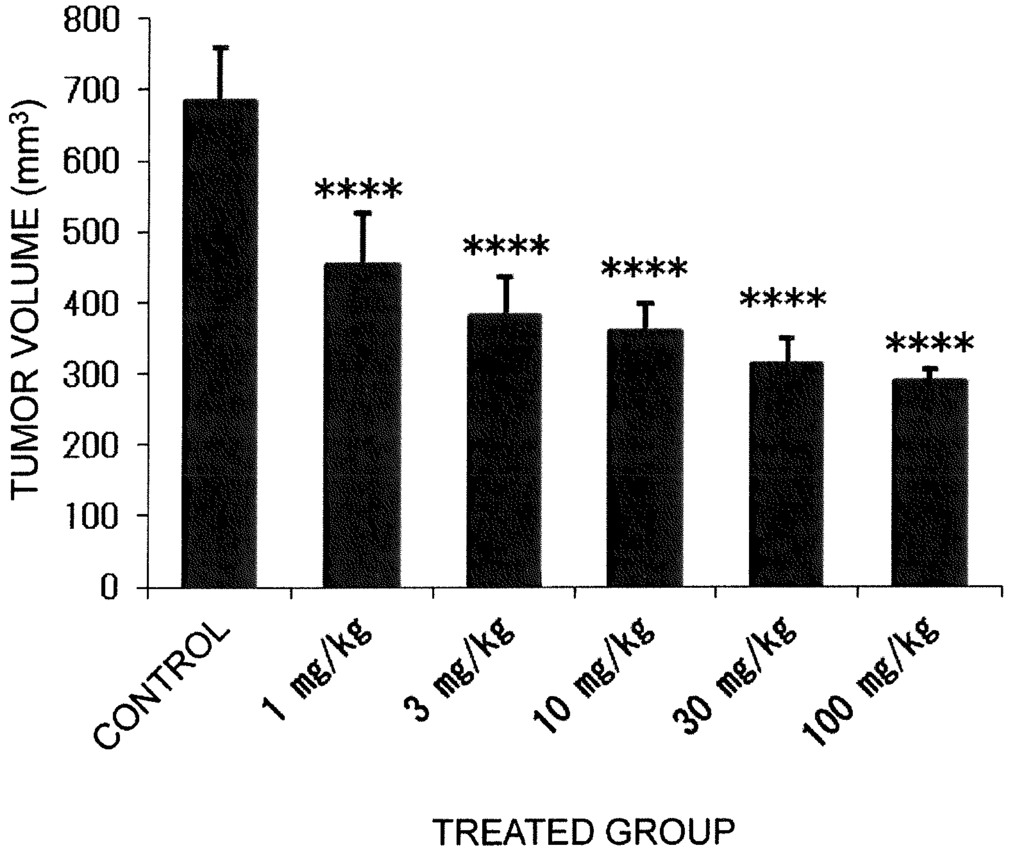
TUMOR VOLUME (mm3)
800
700
600
500
400
300
200
100
0
****
****
****
****
****
CONTROL
1 mg/kg
3 mg/kg
10 mg/kg
30 mg/kg
100 mg/kg
TREATED GROUP

*Fig.5*

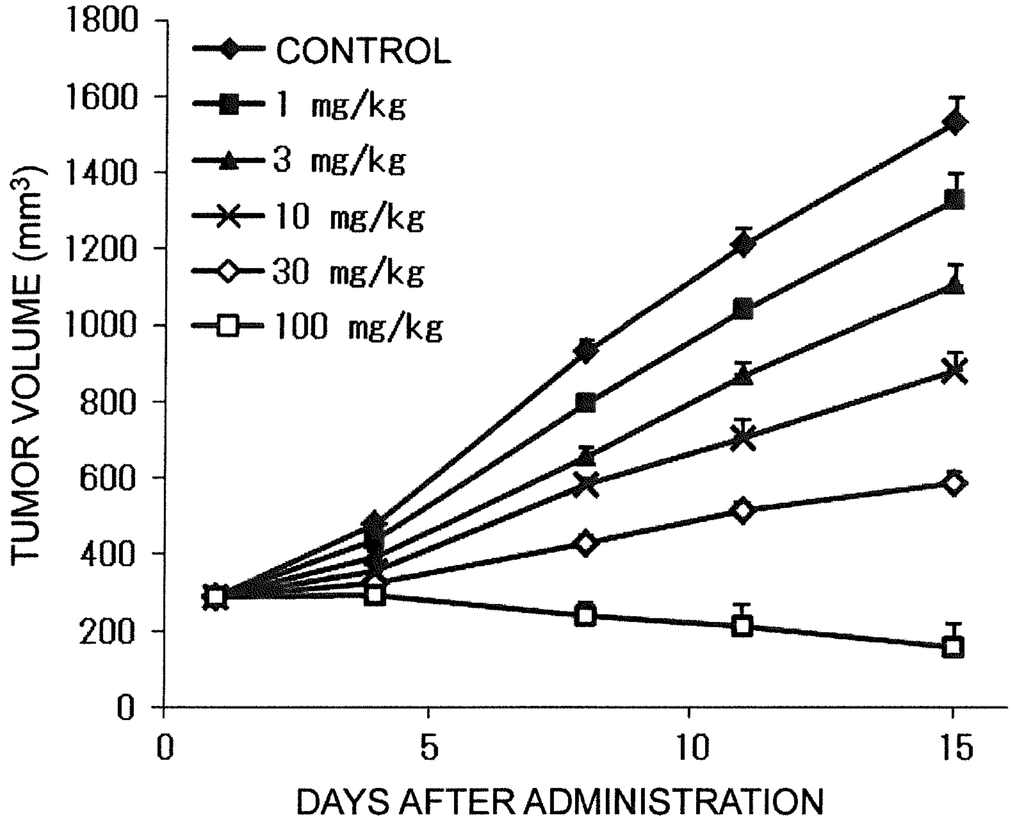
1800
1600
1400
1200
1000
800
600
400
200
0
TUMOR VOLUME (mm3)
CONTROL
1 mg/kg
3 mg/kg
10 mg/kg
30 mg/kg
100 mg/kg
0
5
10
15
DAYS AFTER ADMINISTRATION

*Fig.6*

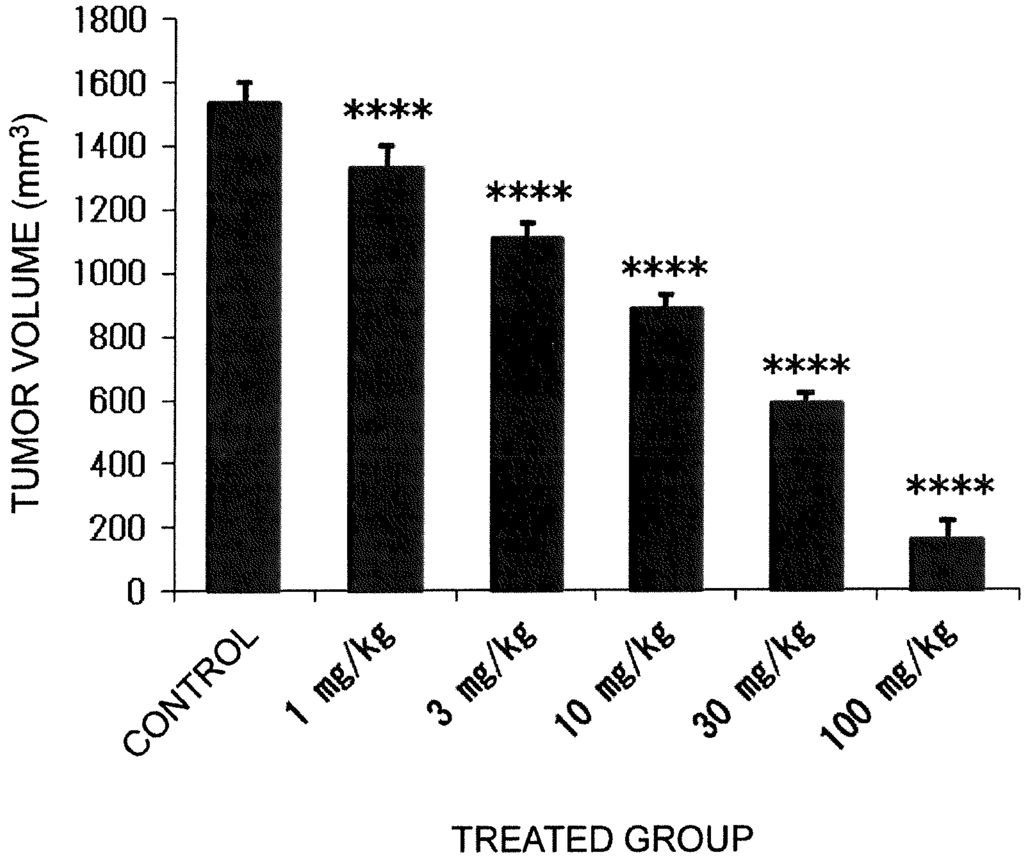
1800
1600
1400
1200
1000
800
600
400
200
0
TUMOR VOLUME (mm3)
****
****
****
****
****
CONTROL
1 mg/kg
3 mg/kg
10 mg/kg
30 mg/kg
100 mg/kg
TREATED GROUP

**INTERNATIONAL SEARCH REPORT**

International application No.
PCT/JP2016/074017

A. CLASSIFICATION OF SUBJECT MATTER
*A61K31/47*(2006.01)i, *A61P1/16*(2006.01)i, *A61P35/00*(2006.01)i, *A61P43/00* (2006.01)i

According to International Patent Classification (IPC) or to both national classification and IPC

B. FIELDS SEARCHED

Minimum documentation searched (classification system followed by classification symbols)
A61K31/47, A61P1/16, A61P35/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2014/113729 A2 (FOUNDATION MEDICINE, INC.), 24 July 2014 (24.07.2014), claims 2, 9; page 1, lines 23 to 31; page 4, lines 10 to 14; page 54, lines 6 to 16 & US 2015/0366866 A1 & EP 2945652 A2 | 1-7,11-15<br>1-15 |
| Y | Takuji OKUSAKA et al., "Chemotherapy for biliary tract cancer", Tando, 2013, 27(1), pages 124 to 134, ISSN 0914-0077, particularly, page 128, column (3), page 130, columns 4, 6, page 132, column 3) | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 September 2016 (26.09.16) | 04 October 2016 (04.10.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/074017

C (Continuation). DOCUMENTS CONSIDERED TO BE RELEVANT

| Category* | Citation of document, with indication, where appropriate, of the relevant passages | Relevant to claim No. |
|---|---|---|
| Y | Hiroyuki TAKAHASHI et al., "Tankangan ni Kekkan Shinsei Sogaiyaku Aruiwa EGFR Sogaiyaku wa Yukoka -Zen Rinsho Shiken karano Kanosei-", The Biliary Tract & Pancreas, 2015.02, 36(2), pages 153 to 160, ISSN 0388-9408, particularly, pages 154 to 155, II, column 2., page 159, column of 'Owarini' | 1-15 |
| Y | Toshiyuki TAMAI et al., "Renvatinib no Kaihatsu Senryaku Oyobi Shokaki Gan no Kaihatsu Jokyo", BIO Clinica, 2014, 29(2), pages 61 to 65, ISSN 0919-8237, particularly, pages 61 to 63, column 1. | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2004053908 A
- US 2004253205 A
- US 2010105031 A
- US 2009209580 A
- US 2009264464 A
- JP 2015162004 A

### Non-patent literature cited in the description


- **LASOTA et al.** Mutations in Exons 9 and 13 of KIT Gene Are Rare Events in Gastrointestinal Stromal Tumors. *American Journal of Pathology,* 2000, vol. 157, 1091-1095
- **BERDEL et al.** Recombinant Human Stem Cell Factor Stimulates Growth of a Human Glioblastoma Cell Line Expressing c-kit Protooncogenel. *Cancer Research,* 1992, vol. 52, 3498-3502
- **LENNARTSSON et al.** The stem cell factor receptor/c-Kit as a drug target in cancer. *Current Cancer Drug Targets,* 2006, vol. 6, 65-75
- **TURNER et al.** Fibroblast growth factor signalling: from development to cancer. *Nature Reviews Cancer,* 2010, vol. 10, 116-129
- **WELLS et al.** Targeting the RET Pathway in Thyroid Cancer. *Clinical Cancer Research,* 2009, vol. 15, 7119-7123
- **JUAN et al.** Cisplatin plus gemcitabine versus gemcitabine for biliary tract cancer. *The New England Journal of Medicine,* 2010, vol. 362, 1273-1281
- **ANG.** Role of the fibroblast growth factor receptor axis in cholangiocarcinoma. *Journal of Gastroenterology and Hepatology,* 2015, vol. 30, 1116-1122